# EUROPÄISCHE PATENTSCHRIFT

(11) **EP 3 967 761 B1**
(45) Veröffentlichungstag und Bekanntmachung des Hinweises auf die Patenterteilung: **15.03.2023**
(21) Anmeldenummer: 20195201.7
(22) Anmeldetag: 09.09.2020
(51) Int. Cl.: C12P 5/02, C12M 1/107, C12M 1/36, C12M 1/00, C02F 3/00, G05B 13/04

(54) **VERFAHREN ZUR ERZEUGUNG EINES METHANANGEREICHERTEN GASES**
METHOD FOR CREATING METHANE-ENRICHED GAS
PROCÉDÉ DE GÉNÉRATION D'UN GAZ ENRICHI EN MÉTHANE

(43) Veröffentlichungstag der Anmeldung: 16.03.2022
(73) Patentinhaber: Hitachi Zosen Inova Schmack GmbH, 92421 Schwandorf (DE)
(72) Erfinder: SCHOLZ, Matthias, 93186 Pettendorf (DE)
(74) Vertreter: Glück Kritzenberger Patentanwälte PartGmbB

(56) Entgegenhaltungen:
- EP-A1- 2 982 740
- WO-A1-2020/152017
- DE-A1-102012 105 658
- DE-A1-102012 112 889
- DE-A1-102014 111 287
- DE-A1-102014 111 298

## Beschreibung

### Technisches Gebiet

Die Erfindung betrifft ein Verfahren zur Erzeugung eines methanangereicherten Gases.

### Stand der Technik

Im Rahmen der Energiewende nehmen die erneuerbaren Energien einen immer größer werdenden Anteil an der erzeugten Energie ein. Da gerade erneuerbare Energieformen wie Strom aus Windkraft oder Photovoltaik sowohl zeitlich wie auch mengenmäßig nicht kontinuierlich und gleichmäßig zur Verfügung stehen und dabei auf Verbraucherseite ebenfalls auf eine zeitlich schwankende Nachfrage treffen, werden Speichermöglichkeiten für erneuerbare Energien zunehmend wichtiger. Methan ist in diesem Zusammenhang von zentraler Bedeutung als chemischer Energieträger mit vielfältigen Einsatzmöglichkeiten in den Bereichen Wärmeerzeugung, Kraftstoffe oder Stromerzeugung.

Insbesondere in Energieumwandlungssystemen, die nach dem "Power-to-Gas"-Prinzip überschüssigen Strom in gasförmige chemische Energieträger umwandeln, erscheint Methan als geeigneter Energieträger, da es im Gegensatz zu Wasserstoff bei der vorhandenen Infrastruktur durch das Erdgasnetz sowie dem bereits bestehenden Netz an Erdgastankstellen eine gut speicherbare Energieform darstellt. Methan wird dabei aus den Ausgangsstoffen Kohlendioxid und Wasserstoff erzeugt. Der Wasserstoff wird in diesen Systemen in der Regel durch Elektrolyse von Wasser mit Hilfe eines Elektrolyseurs zur Verfügung gestellt, wobei Überschussstrom oder anderer günstiger Strom verwendet wird. Das notwendige Kohlendioxid für die Methanerzeugung kann aus verschiedenen Quellen wie Industrie- oder Verbrennungsabgasen stammen, bevorzugt wird jedoch klimafreundliches CO₂ aus erneuerbaren Quellen wie Biomasse verwendet, wie es etwa in Biogasanlagen oder auch als Faulgas in Kläranlagen anfällt.

Verfahren zur biologischen Methanisierung, bei denen das Biomethan durch methanogene Mikroorganismen gebildet wird, kommen im Vergleich zur chemisch katalytischen Methanisierung, beispielsweise nach dem Sabatier-Verfahren, ohne teure und empfindliche Katalysatoren aus und stellen geringere Anforderungen an Reaktionsbedingungen wie Temperatur und Druck sowie an die Reinheit der Ausgangsgase CO₂ und H₂.

Beispielsweise beschreibt die WO 2008/094282 A1 ein biologisches System zur Methanproduktion aus Wasserstoff und Kohlendioxid unter Verwendung einer Mikroorganismenkultur in einem Kulturmedium. Das Kohlendioxid stammt dabei aus einem industriellen Prozess, während der Wasserstoff u.a. durch Elektrolyse unter Einsatz von billigem Überschussstrom gewonnen wird.

Ein System zur Speicherung von elektrischer Energie in Form von Methan ist aus der WO 2012/110257 A1 bekannt, wobei Strom aus erneuerbaren und nicht erneuerbaren Energien zur Wasserstoffproduktion verwendet wird. Der Wasserstoff wird zusammen mit Kohlendioxid in einen Reaktor eingeleitet, der Kulturen von methanogenen Mikroorganismen in einem Kulturmedium enthält, welche dann Methan produzieren. Dabei wird das Partialdruckverhältnis der Gase Wasserstoff zu Kohlendioxid im Reaktionsbehälter während der Zellwachstumsphase auf einem Wert kleiner oder gleich 4:1 und in der Methanbildungsphase auf einem Wert 5:1 oder höher eingestellt.

Aus der DE 10 2012 112 889 A1 ist ein Energieumwandlungssystem mit einer Elektrolyseeinheit zur elektrochemischen Erzeugung von Wasserstoff und Sauerstoff aus Wasser und einem anaeroben Bioreaktor bekannt. Das Energieumwandlungssystem umfasst eine Steuer- und Regelungseinheit zur Steuerung und Regelung von sowohl der Zufuhr von Wasserstoff in den Bioreaktor als auch der Entnahme des methanhaltigen Produktgases aus dem Bioreaktor. Die Steuerungs- und Regelungseinheit verwendet als primäre Regelgröße den CO₂-Gehalt des methanhaltigen Biogases, welcher auf einen Wert von Null geregelt werden soll. Solange Kohlendioxid in dem methanhaltigen Biogas vorhanden ist, erfolgt eine positive Rückkopplung auf den Zufluss von Wasserstoff in den anaeroben Bioreaktor. Ist kein CO₂ mehr im Biogas vorhanden, wird die Wasserstoffzufuhr in den anaeroben Bioreaktor abgeregelt und der mit dem Elektrolyseur produzierte Wasserstoff wird zwischengespeichert.

Aus der EP 2 982 740 A1 ist ein Verfahren zur Erzeugung von Methan aus einem kohlendioxidhaltigen Biogas und Wasserstoff in einem Bioreaktor bekannt. Eine Steuer- und Regelungstechnik steuert und regelt dabei sowohl die Zu- und Abflüsse an Methanisierungsmedium sowie die Gaszu- und -abflüsse in den und aus dem Bioreaktor, insbesondere auch das Verhältnis der Gasflüsse von zugegebenem Wasserstoff im Verhältnis zu zugegebenem CO₂-haltigen Gas. In der Regel wird H₂ zu CO₂ in einem Verhältnis von 4:1 eingesetzt, eine Feinregelung erfolgt jedoch nach der Zusammensetzung des Ausgangsgases aus dem Bioreaktor. Um einen möglichst hohen Biomethangehalt zu erreichen, darf weder zu viel Wasserstoff noch zu viel CO₂ im Ausgangsgas des Bioreaktors messbar sein. Entsprechend erfolgt eine Feinregelung, bei der das Verhältnis von zugegebenem H₂ zu CO₂ von dem Verhältnis von 4:1 nach oben oder unten etwas abweichen kann.

In der weit überwiegenden Zahl der im Stand der Technik beschriebenen Verfahren erfolgt die biologische Methanisierung bei einem Verhältnis der Eduktgase H₂ zu CO₂ von 4:1, so dass gemäß der Reaktionsgleichung der Methanisierungsreaktion

4 H₂ + CO₂ → CH₄ + 2 H₂O

kein Eduktgas im Überschuss vorliegt.

Ziel jedes technischen Verfahrens zur Erzeugung eines methanangereicherten Gases ist es, einen möglichst hohen Methangehalt im Produktgas zu erreichen und die Anteile an den nicht umgesetzten Eduktgasen Wasserstoff und Kohlendioxid zu minimieren. Insbesondere bei einer Einspeisung des methanangereicherten Gases in ein öffentliches Gasnetz gibt es in der Regel vorgegebene Einspeisehöchstgrenzen für Gasbestandteile wie Wasserstoff und Kohlendioxid, die neben Methan nur in geringen Anteilen von wenigen Prozent enthalten sein dürfen (in Deutschland ist dies beispielsweise festgelegt in den DVGW-Richtlinien der technischen Regeln G 260 und G 262). Dabei liegt die Einspeisegrenze für Wasserstoff niedriger als die für Kohlendioxid.

Da Wasserstoff in der Regel durch Elektrolyse erzeugt werden muss, während Kohlendioxid häufig als Restgas aus anderen Quellen wie Klärgas oder Rohbiogas zur Verfügung steht, stellt der Wasserstoff das kostenintensivere Eduktgas dar.

Der Wasserstoffgehalt des Produktgases stellt also sowohl im Hinblick auf die Einspeisungsfähigkeit des methanangereicherten Gases in ein öffentliches Gasnetz wie auch im Hinblick auf die ökonomische Optimierung des Prozesses den kritischen Wert dar. Aus diesen Gründen steht bei der Regelung des Methanisierungsprozesses nach der Produktgaszusammensetzung der Wasserstoffgehalt des Produktgases im Vordergrund und nicht dessen Kohlendioxidgehalt.

Trotz der beschriebenen Lösungsansätze wird weiterhin nach Verfahren zur biologischen Methanisierung gesucht, mit denen in effizienter und ökonomischer Weise unter Verwendung von Wasserstoff und CO₂ in einem stabilen Prozess ein methanhaltiges Gas mit guter Produktgasqualität hergestellt werden kann.

### Darstellung der Erfindung

Der Erfindung, wie sie in den Ansprüchen gekennzeichnet ist, liegt die Aufgabe zu Grunde, ein effizientes und ökonomisches Verfahren zur biologischen Methanisierung bereitzustellen, das geeignet ist, unter Verwendung von CO₂ und Wasserstoff in einem stabilen Prozess ein methanhaltiges Gas mit guter Produktgasqualität herzustellen.

Diese Aufgabe wird erfindungsgemäß durch das Verfahren zur Erzeugung eines methanangereicherten Gases gemäß Anspruch 1 gelöst. Weitere vorteilhafte Details, Aspekte und Ausgestaltungen der vorliegenden Erfindung ergeben sich aus den abhängigen Ansprüchen, der Beschreibung und der Figur.

Die vorliegende Erfindung stellt ein Verfahren zur Erzeugung eines methanangereicherten Gases zur Verfügung umfassend die Schritte
a) Bereitstellen eines Bioreaktors aufweisend
   - zumindest eine Vorrichtung für die Zufuhr eines Gases,
   - zumindest einen Auslass für die Entnahme des in dem Bioreaktor entstehenden methanangereicherten Gases,
b) Bereitstellen einer Vorrichtung zur Bestimmung des Anteils an Kohlendioxid in dem aus dem Bioreaktor entnommenen methanangereicherten Gas,
c) Festlegung eines Sollwerts S für den Anteil an Kohlendioxid in dem aus dem Bioreaktor entnommenen methanangereicherten Gas,
d) Zufuhr von kohlendioxidhaltigem Gas in den Bioreaktor,
e) Zufuhr von wasserstoffhaltigem Gas in den Bioreaktor,
f) Bildung von methanangereichertem Gas in dem Bioreaktor,
g) Entnahme des in dem Bioreaktor gebildeten methanangereicherten Gases aus dem Bioreaktor,
h) Bestimmen eines Istwertes für den Anteil an Kohlendioxid in dem aus dem Bioreaktor entnommenen methanangereicherten Gas,
i) Vergleich des in Schritt c) festgelegten Sollwerts S mit dem in Schritt h) bestimmten Istwert,
j) Regelung der Menge des in Schritt d) zugeführten kohlendioxidhaltigen Gases und/oder Regelung der Menge des in Schritt e) zugeführten wasserstoffhaltigen Gases derart, dass der in Schritt h) bestimmte Istwert dem in Schritt c) festgelegten Sollwert S entspricht,
wobei der in Schritt c) festgelegte Sollwert S für den Anteil an Kohlendioxid in dem aus dem Bioreaktor entnommenen methanangereicherten Gas der Bedingung 0 Vol. % < S ≤ 5 Vol. % genügt.

Das erfindungsgemäße Verfahren wird mit Hilfe eines Bioreaktors zur Erzeugung eines methanangereicherten Gases mittels biologischer Methanisierung durchgeführt. Der Bioreaktor kann in Form verschiedener Reaktortypen wie beispielsweise Rührkesselreaktor, Säulenreaktor, Rohrreaktor, Blasensäule, Rieselbettreaktor oder auch als Reaktorkaskade ausgebildet sein. Er sollte aus druckfestem und temperaturbeständigen Material gefertigt sein, beispielsweise aus Edelstahl. Im Vergleich zu den anaeroben Fermentern einer Biogasanlage oder zu einem Faulturm einer Kläranlage ist das Reaktorvolumen des Bioreaktors zur biologischen Methanisierung deutlich kleiner, beispielsweise in einem Bereich von 1/10 bis zu 1/1000 des Volumens eines Fermenters einer Biogasanlage oder eines Faulturms einer Kläranlage.

Dieser Bioreaktor wird mit einem kohlendioxidhaltigen Gas sowie mit einem extern erzeugten wasserstoffhaltigen Gas gespeist. Der für die biologische Methanisierung erforderliche Wasserstoff wird bevorzugt durch einen Elektrolyseur erzeugt. Bevorzugt wird der Wasserstoff in dem Elektrolyseur unter Verwendung von Überschussstrom aus dem Stromnetz durch Elektrolyse von Wasser erzeugt. Die Bereitstellung von Überschusstrom übernimmt üblicherweise eine Regelenergiebox, so dass die Anlage zur biologischen Methanisierung in Höhe der Leistung des entsprechenden Elektrolyseurs am Regelenergiemarkt teilnehmen kann. Auch wenn der Elektrolyseur nicht am Regelenergiemarkt teilnimmt, kann anderweitig günstiger Strom (z.B. aus speziellen Stromtarifen) verwendet werden. Alternativ kann auch Wasserstoff aus anderen Quellen wie Hydrolysegas, Synthesegas, Produktgas aus chemischen Reaktionen, H₂ biologischen Ursprungs wie z.B. durch Algen produziertes H₂ oder H₂ aus Photokatalyse zur biologischen Methanisierung eingesetzt werden.

Der Bioreaktor enthält ein wässriges Reaktionsmedium, das als Substrat bzw. Nährmedium für die methanogenen Mikroorganismen dient und damit die Funktion eines Methanisierungsmediums erfüllt. Als Methanisierungsmedium, in dem die Methanisierungsreaktion abläuft, wird entweder ein synthetisches Kulturmedium für methanogene Mikroorganismen verwendet wie im Stand der Technik beschrieben (z.B. WO 2008/094282 A1, EP 2 982 740 A1) oder es wird ein komplexes Nährmedium auf der Grundlage von Biomasse eingesetzt, welches im Wesentlichen aus einem Gärrest als Substrat besteht und gegebenenfalls durch Nährstoffe und/oder Spurenelemente ergänzt wird (z.B. EP 2 982 740 A1). Als Substrat kann beispielsweise der Gärrest aus einer Biogasanlage, insbesondere der Gärrest aus einer späteren Gärstufe oder einem Nachgärer eingesetzt werden, wobei der Gärrest auch aus einem Endlager stammen kann. Ebenso eignen sich als Substrat für den Bioreaktor zur biologischen Methanisierung prinzipiell jede Art von Rohschlamm und/oder Faulschlamm aus einer Kläranlage. Methanogene Mikroorganismen, insbesondere hydrogenotrophe methanogene Mikroorganismen werden einem synthetischen Kulturmedium in Form einer Reinkultur oder einer Mischkultur von geeigneten Mikroorganismen zugesetzt. Bei einem Gärrest als Substrat sind in der Regel bereits methanogene Mikroorganismen vorhanden, es können jedoch optional zusätzlich hydrogenotrophe methanogene Mikroorganismen hinzugegeben werden. Besonders geeignet sind thermophile Methanogene der Gattung *Methanothermobacter.*

Der Bioreaktor zur biologischen Methanisierung weist vorteilhafterweise ein geeignetes System zur Gaseinbringung in das flüssige Reaktormedium für das CO₂-haltige Gas sowie für das wasserstoffhaltige Gas auf. Das CO₂-haltige Gas sowie das wasserstoffhaltige Gas werden bevorzugt über Zuführleitungen in den Bioreaktor eingebracht. Vorzugsweise werden die beiden Gase bereits vor der Einbringung in den Reaktor in einer Gasmischkammer gemischt und anschließend über eine gemeinsame Eduktgasleitung in den Reaktor geleitet. Die Zuführung der H₂- und CO₂-haltigen Eduktgase erfolgt entweder direkt über mindestens eine Zufuhrleitung in den Bioreaktor, bevorzugt im unteren Bereich des Bioreaktors, oder über eine Gaseinbringung in die Substratzufuhrleitung für den Bioreaktor.

Als besonders geeignete Systeme zur Gaseinbringung haben sich Rührwerke, insbesondere Begasungsrührwerke und Kaskadenrührwerke (z.B. Ekato), dynamische Mischer, Mehrphasenpumpen (z.B. Edur-Pumpe) sowie Säulenreaktoren erwiesen. Diese Gaseinbringsysteme können mit im Reaktor integrierten Systemen zur Gasfeinverteilung wie Lochplatten, Diffusoren, Sinterwerkstoffen, Membranen kombiniert werden. Damit die Gaseinbringung und -verteilung technisch gut funktioniert, sollte die Viskosität des Fermenterinhalts nicht zu hoch sein.

Das gebildete methanangereicherte Gas wird über eine Produktgasleitung, die vorzugsweise am oberen Ende des Bioreaktors angeordnet ist, aus dem Bioreaktor abgeführt. In der Regel erfolgt vor einer weiteren Verwertung des Produktgases eine Gasaufbereitung mit den Schritten Gastrocknung und Gaskühlung sowie gegebenenfalls eine Entschwefelung, eine Gaswäsche zur Abtrennung weiterer Komponenten wie z.B. Ammoniak und/oder eine H₂-Abtrennung. Aus dem methanangereicherten Gas, das als Produktgas den Bioreaktor verlässt, wird auf diese Weise ein aufbereitetes SNG (Substitute Natural Gas) bzw. Bioerdgas oder Biomethan, das in ein öffentliches Gasnetz eingespeist werden kann. Neben der Einspeisung von SNG ins Erdgasnetz besteht die Möglichkeit einer Nutzung als Kraftstoff beispielsweise in Form einer Bioerdgastankstelle oder eine stoffliche Nutzung. Als weitere Verwertungsmöglichkeit bietet sich in Zeiten eines zu geringen Stromangebots eine Rückverstromung von Biomethan mit gleichzeitiger Wärmeerzeugung, beispielsweise über ein BHKW an. In diesem Fall wird ein Zwischenspeicher für das produzierte Biomethan benötigt. Dieser wie auch ein BHKW sind jedoch häufig bereits in entsprechenden Anlagen integriert und können so in vorteilhafter Weise für das erfindungsgemäße Verfahren genutzt werden.

Der Bioreaktor ist vorteilhafterweise zudem ausgestattet mit einem Druckhalteventil und entsprechend druckstabilen Komponenten, so dass ein Reaktionsdruck höher als der Atmosphärendruck, insbesondere ein Überdruck von bis zu 30 bar, bevorzugt ein Überdruck von bis zu 16 bar eingestellt werden kann. Der Bioreaktor ist optional mit Sensoren zur Messung von Temperatur, Druck und pH-Wert ausgestattet. Zusätzlich kann ein System zur pH-Regelung bzw. zur Zudosierung von Lauge oder Säure vorgesehen sein.

Erfindungsgemäß wird die Zudosierung der Eduktgase H₂ (bzw. wasserstoffhaltiges Gas) und CO₂ (bzw. CO₂-haltiges Gas wie Rohbiogas oder Klärgas) nach dem CO₂-Gehalt im Produktgas geregelt. Dabei erfolgt zunächst die Festlegung eines Sollwerts S für den Anteil an Kohlendioxid in dem aus dem Bioreaktor entnommenen methanangereicherten Gas. Die Regelung der Menge des zugeführten kohlendioxidhaltigen Gases und/oder die Regelung der Menge des zugeführten wasserstoffhaltigen Gases erfolgt erfindungsgemäß derart, dass der gemessene Istwert des Anteils an CO₂ im Produktgas dem festgelegten Sollwert S möglichst nahe kommt oder diesem entspricht. Bevorzugt wird bei allen Gasströmen von und zu dem Bioreaktor sowohl die Gaszusammensetzung als auch der Gasfluss (Gasmenge pro Zeit) gemessen.

Es wird also der CO₂-Gehalt im Produktgas auf einen bestimmten Wert geregelt. Wie nachfolgend noch näher beschrieben beträgt dieser Wert für den CO₂-Gehalt im Produktgas bevorzugt 1 Vol. %, besonders bevorzugt liegt der Wert im Bereich von 0,1 Vol. % bis 1,5 Vol. %. Im Gegensatz zu den aus dem Stand der Technik bekannten Verfahren besteht das Ziel also gerade nicht darin, einen CO₂-Gehalt von Null im Produktgas zu erreichen. Bei der Regelung wird als Stellgröße die Menge an H₂ und/oder die Menge an CO₂ im Eduktgasstrom variiert, in jedem Fall also das Verhältnis von H₂ zu CO₂ im Eduktgasstrom verändert, und zwar derart, dass der vorgegebene Sollwert S erreicht wird. Für den Eduktgasstrom wird in der Regel entweder ein bestimmter Wert für den Volumenstrom an wasserstoffhaltigem Gas festgelegt und der Volumenstrom für das CO₂ enthaltende Gas wird variiert oder der Eduktgasstrom wird mit einem festgelegten Volumenstrom an CO₂ enthaltendem Gas gespeist und der Volumenstrom an wasserstoffhaltigem Gas wird entsprechend variiert.

Über die Regelung des CO₂-Gehalts im Produktgas kann dafür gesorgt werden, dass im Produktgas stabil ein niedriger CO₂-Gehalt in der Größenordnung bis zu wenigen Prozent CO₂ vorhanden ist, was für eine gute Gasqualität wichtig ist.

Zudem wurde festgestellt, dass die CO₂-Konzentration im Produktgas in erheblichem Maß den pH-Wert im Bioreaktor beeinflusst. Bekanntermaßen funktioniert die biologische Methanisierung in einem pH-Bereich von ca. 6,5 bis etwa 9,0 mit einem bevorzugten Bereich zwischen pH 7,0 und 8,0 und insbesondere bevorzugt zwischen pH 7,0 und 7,5. Aus diesem Grund wird bei den aus dem Stand der Technik bekannten Verfahren häufig eine pH-Regelung über eine pH-Messung mit einer pH-Sonde direkt im Reaktor und einer entsprechenden anschließenden Zudosierung von Säure oder Lauge je nach gemessenem pH-Wert durchgeführt. Der pH-Wert wird dabei also über Zugabe von Säure oder Lauge direkt in Richtung des gewünschten Bereichs verändert.

Bei Anwendung des erfindungsgemäßen Verfahrens wird der pH-Wert im Bioreaktor durch die CO₂-Konzentration im Produktgas beeinflusst. Dabei führt ein zu geringer Anteil an CO₂ im Produktgas zu einem Anstieg des pH-Wertes im Bioreaktor, während ein zu hoher Anteil an CO₂ im Produktgas zu einer Versäuerung des Reaktorinhalts führt. Die dafür zugrunde liegenden physikalisch-chemischen Vorgänge werden im Henry-Gesetz und in der Henderson-Hasselbalch-Gleichung beschrieben. Durch die Regelung des CO₂-Gehalts im Produktgas wird also eine Stabilisierung des pH-Wertes im Reaktionsmedium erreicht, ohne dass hierfür eine externe Zudosierung von Säure oder Lauge erfolgen muss. Der Bioreaktor sollte trotzdem einen Anschluss einer Dosierpumpe für die Zugabe von Lauge oder Säure aufweisen. Eine solche Dosierpumpe dient aber nicht in erster Linie einer kontiuierlichen pH-Regelung, sondern dem Gegensteuern bei Störfällen, einer Stabilisierung eines Methanisierungsmediums mit geringer Pufferkapazität o.ä.

Die genannten Effekte ergänzen sich zudem, da die durch die Regelung des Methanisierungsprozesses über den CO₂-Gehalt im Produktgas bewirkte Stabilisierung des pH-Wertes des Reaktionsmediums im Bioreaktor zu einem stabilen Prozess führt, welcher zudem mit einer guten Gasqualität einhergeht, da der CO₂-Gehalt im Produktgas auf einen niedrigen Wert begrenzt wird. Erstaunlicherweise kann damit auf eine pH-Regelung mit pH-Sonde im Bioreaktor und entsprechender Zudosierung von Säure oder Lauge verzichtet werden, wodurch ein einfacheres und kostengünstigeres Verfahren zur Verfügung steht. Eine Vorrichtung zur Messung des CO₂-Gehaltes des Produktgases sowie entsprechende Durchflussregler für eine dosierte Zugabe der Eduktgase H₂ und CO₂ sind in den üblichen Anlagen zur Methanisierung ohnehin vorhanden und können damit in vorteilhafter Weise genutzt werden.

Bei den aus dem Stand der Technik bekannten Verfahren, bei denen das Verhältnis von H₂ zu CO₂ auf einen Wert von 4:1 (oder auch auf andere festgelegte Werte) geregelt wird, ergibt sich häufig das Problem, dass sich negativ auf den Prozess der biologischen Methanisierung auswirkende Störgrößen (z.B. fehlende Nährstoffe, Sauerstoffeintrag, Störstoffe, Änderung des pH-Wertes) zu einer Verschlechterung der Qualität des Methanisierungsgases führen, welche sich darin äußert, dass der Wasserstoffgehalt im Produktgas ansteigt, weil der als Eduktgas zugegebene Wasserstoff nicht mehr komplett umgesetzt wird. Bei einem Verhältnisregler würde mit steigenden H₂-Gehalt im Produktgas auch der CO₂-Gehalt im Produktgas ansteigen, weil mit steigendem Anteil an nicht umgesetzten Wasserstoff auch der Anteil an nicht umgesetztem Kohlendioxid zunimmt. Dies führt zu einem Absinken des pH-Wertes im Bioreaktor und damit zu einer weiteren Verschlechterung der Gasqualität. Bei der erfindungsgemäßen Regelung nach dem Kohlendioxid-Gehalt im Produktgas wird das aktuelle Verhältnis von H₂ zu CO₂ im Produktgas nicht beachtet, wodurch wie bereits beschrieben ein stabiler pH-Wert erreicht wird, weil der CO₂-Gehalt im Produktgas unabhängig vom H₂-Gehalt eingestellt wird.

Als Regelgröße des erfindungsgemäßen Verfahrens dient also der CO₂-Gehalt des Produktgases. Dieser wird bevorzugt kontinuierlich gemessen und in Vol. % als Istwert in die Regelung rückgeführt. Als Führungsgröße bzw. Sollwert wird ein bestimmter Wert S für den Anteil an Kohlendioxid in dem aus dem Bioreaktor entnommenen methanangereicherten Gas vorgegeben (Vol. %). Erfindungsgemäß muss S die Bedingung 0 Vol. % < S ≤ 5 Vol. % erfüllen.

Alternativ zum Volumenanteil des Kohlendioxids könnte auch der Stoffmengenanteil als Regelgröße dienen. Bei einer Messung des Massenanteils an Kohlendioxid müsste dieser im Verhältnis zu den anderen Bestandteilen des Produktgases auf den Stoffmengenanteil umgerechnet werden.

Bevorzugt genügt der Sollwert S für den Anteil an Kohlendioxid in dem aus dem Bioreaktor entnommenen methanangereicherten Gas der Bedingung 0 Vol. % < S ≤ 4 Vol. %, besonders bevorzugt der Bedingung 0 Vol. % < S ≤ 3 Vol. %, insbesondere bevorzugt der Bedingung 0 Vol. % < S ≤ 2 Vol. % und ganz besonders bevorzugt der Bedingung 0 Vol. % < S ≤ 1,5 Vol. %.

Gemäß weiteren bevorzugten Ausführungsformen der vorliegenden Erfindung erfüllt der der Sollwert S für den Anteil an Kohlendioxid in dem aus dem Bioreaktor entnommenen methanangereicherten Gas die Bedingung 0,1 Vol. % ≤ S ≤ 5 Vol. %, bevorzugt die Bedingung 0,1 Vol. % ≤ S ≤ 4 Vol. %, besonders bevorzugt die Bedingung 0,1 Vol. % < S ≤ 3 Vol. %, insbesondere bevorzugt die Bedingung 0,1 Vol. % ≤ S ≤ 2 Vol. %, und ganz besonders bevorzugt die Bedingung 0,1 Vol. % ≤ S ≤ 1,5 Vol. %.

Gemäß weiteren bevorzugten Ausführungsformen der vorliegenden Erfindung erfüllt der der Sollwert S für den Anteil an Kohlendioxid in dem aus dem Bioreaktor entnommenen methanangereicherten Gas die Bedingung 0,2 Vol. % ≤ S ≤ 5 Vol. %, bevorzugt die Bedingung 0,2 Vol. % ≤ S ≤ 4 Vol. %, besonders bevorzugt die Bedingung 0,2 Vol. % < S ≤ 3 Vol. %, insbesondere bevorzugt die Bedingung 0,2 Vol. % ≤ S ≤ 2 Vol. %, und ganz besonders bevorzugt die Bedingung 0,2 Vol. % ≤ S ≤ 1,5 Vol. %.

Gemäß weiteren bevorzugten Ausführungsformen der vorliegenden Erfindung erfüllt der der Sollwert S für den Anteil an Kohlendioxid in dem aus dem Bioreaktor entnommenen methanangereicherten Gas die Bedingung 0,5 Vol. % ≤ S ≤ 5 Vol. %, bevorzugt die Bedingung 0,5 Vol. % ≤ S ≤ 4 Vol. %, besonders bevorzugt die Bedingung 0,5 Vol. % < S ≤ 3 Vol. %, insbesondere bevorzugt die Bedingung 0,5 Vol. % ≤ S ≤ 2 Vol. %, und ganz besonders bevorzugt die Bedingung 0,5 Vol. % ≤ S ≤ 1,5 Vol. %.

Gemäß einer besonders bevorzugten Ausführungsform der vorliegenden Erfindung beträgt der Sollwert S für den Anteil an Kohlendioxid in dem aus dem Bioreaktor entnommenen methanangereicherten Gas rund 1 Vol. %.

Um die Regelung technisch durchzuführen, wird die Qualität des Produktgases nach Verlassen des Methanisierungsreaktors bestimmt. Vorrichtungen zur Messung des CO₂-Gehaltes des Produktgases (Regelgröße) sowie des CH₄-Gehalts und des H₂-Gehalts des Produktgases sind in einem System zur Methanisierung in der Regel ohnehin vorhanden und können so in vorteilhafter Weise genutzt werden. Vorzugsweise befindet sich die Vorrichtung zur Produktgasanalyse direkt im Anschluss an den Methanisierungsreaktor oder im Bereich der Gasaufbereitung. Auf diese Weise wird der Istwert des Anteils an Kohlendioxid in dem aus dem Bioreaktor entnommenen methanangereicherten Gas fortlaufend gemessen. Anschließend wird rechnerisch die Regelabweichung zwischen Soll- und Istwert bestimmt, indem die Differenz zwischen Sollwert und Istwert gebildet wird.

Besonders bevorzugt erfolgt die Regelung der Menge des zugeführten kohlendioxidhaltigen Gases und/oder die Regelung der Menge des zugeführten wasserstoffhaltigen Gases ausschließlich auf Basis des Vergleichs von festgelegtem Sollwert S mit dem gemessenen Istwert des Anteils an Kohlendioxid in dem aus dem Bioreaktor entnommenen methanangereicherten Gas. Es werden in diesem Fall also keine weiteren Parameter wie Gaszusammensetzung des Produktgases oder Druck, Temperatur, Redoxpotential oder pH-Wert im Bioreaktor berücksichtigt.

Der pH-Wert stellt einen geeigneten Parameter dar, um bei bestimmten vorherrschenden Reaktionsbedingungen (z.B. Druck, Temperatur) im Bioreaktor den jeweiligen Wert für den Sollwert S festzulegen und/oder im Laufe der Methanisierung anzupassen. Prinzipiell werden möglichst kleine CO₂-Gehalte im Produktgas (beispielsweise weniger als 2 Vol. %) bevorzugt, da auf diese Weise ein maximaler Methananteil im Produktgas erreicht werden kann. Andererseits sind sehr kleine CO₂-Gehalte von beispielsweise weniger als 0,5 Vol. % mess- und regelungstechnisch vergleichsweise anspruchsvoll darzustellen. Inwiefern unterschiedliche Vorgaben für den Sollwert S den pH-Wert des Methanisierungsmedium im Bioreaktor beeinflussen, hängt auch von der Beschaffenheit des verwendeten Substrates ab. Zum einen beeinflusst die Pufferkapazität des Methanisierungsmediums die Wahl des Sollwertes. Ein synthetisches Kulturmedium mit wenig Pufferkapazität reagiert anders auf einen hohen Sollwert S als ein Methanisierungsmedium auf Grundlage eines Gärrestes mit einer komplexen Zusammensetzung. Methanisierungsmedien, die ausgehend von Gärresten oder Faulschlämmen adaptiert werden, können beispielsweise in der Ausgangszusammensetzung pH-Werte von pH 7,5 oder höher aufweisen. In diesem Fall wird der vorgegebene Sollwert S für den CO₂-Gehalt auf einen höheren Wert, beispielsweise 1,5 Vol. % oder höher festgelegt, damit durch den höheren CO₂-Gehalt einem zu hohen Anstieg des pH-Wertes entgegengewirkt wird.

Der Regler ermittelt in jedem Fall gemäß den vorgegebenen Regelparametern als Stellgröße ein bestimmtes Verhältnis von H₂ zu CO₂ im Eduktgas, welches über die Regelstrecke, also die biologische Methanisierung im Methanisierungsreaktor, auf die Regelgröße einwirkt. Je nachdem, ob während der Methanisierung der Eduktgasvolumenstrom für H₂ (bzw. H₂-haltiges Gas) oder CO₂ (bzw. CO₂-haltiges Gas) konstant gehalten werden soll, wird zur Einstellung der Stellgröße der Volumenstrom an H₂ (bzw. an H₂-haltigem Gas) oder an CO₂ (bzw. CO₂-haltigem Gas) variiert. Es können aber auch beide Eduktgasströme gleichzeitig oder aufeinanderfolgend variiert werden.

Besonders bevorzugt handelt es sich bei der Vorrichtung für die Zufuhr eines kohlendioxidhaltigen Gases und/oder bei der Vorrichtung für die Zufuhr eines wasserstoffhaltigen Gases um eine Vorrichtung zur Durchflussregelung. Um die jeweils aktuelle Vorgabe des Reglers für die Stellgröße technisch umsetzen zu können, werden bevorzugt Durchflussregler eingesetzt, die eine variable Zugabe der Volumenströme der Eduktgase H₂ und CO₂ in den Methanisierungsreaktor gewährleisten.

Gemäß einer bevorzugten Ausführungsform wird der Vergleich des festgelegten Sollwerts S mit dem gemessenen Istwert sowie die Regelung der Menge des zugeführten kohlendioxidhaltigen Gases und/oder die Regelung der Menge des zugeführten wasserstoffhaltigen Gases durch eine Rechnereinheit durchgeführt. Bei dem "Regler" an sich handelt es also sich bevorzugt um einen Algorithmus, der als Softwarelösung innerhalb einer geeigneten Umgebung implementiert wird (z.B. SPS, ibaLogic). Diese Programmvorschrift muss jedoch auf einer geeigneten Hardware implementiert werden, die mit der Methanisierungsanlage in kommunikativer Verbindung steht. Dies kann beispielsweise ein geeigneter Computer sein, der zur Steuerung und Regelung der Anlage benutzt wird. Dieser muss nicht zwangsweise am Methanisierungsreaktor stehen, sondern kann auch über Leitungen oder Remote Control mit dieser verbunden sein.

Der Methanisierungsreaktor selbst stellt die Regelstrecke des Systems dar. Als auf die Regelstrecke einwirkende Störgrößen können in dem System alle Parameter fungieren, die eine Änderung der Zusammensetzung des Produktgases der Methanisierung bewirken, also z.B. Änderungen des pH-Wertes oder der Temperatur im Bioreaktor, aber auch Änderungen in der Zusammensetzung des Reaktormediums aufgrund von Wachstum und Stoffwechsel der Mikroorganismen.

Ein entscheidender Vorteil des erfindungsgemäßen Verfahrens im Vergleich zu anderen im Stand der Technik bekannten Verfahren ist, dass die Wirtschaftlichkeit erhöht wird, weil in nicht unerheblichem Maße schon vorhandene Infrastruktur und Technik benutzt wird, um die Methanausbeute durch den Bioreaktor zur biologischen Methanisierung zu erhöhen. Ein zusätzlicher Vorteil besteht darin, dass mit dem erfindungsgemäßen Verfahren gleichzeitig mit einer Regelung auf eine gute Produktgasqualität auch eine Einstellung eines stabilen pH-Wertes erfolgt, ohne dass in erheblichen Mengen Lauge oder Säure zudosiert werden muss. Die Regelung des Kohlendioxidgehaltes des Produktgases übernimmt somit im Wesentlichen auch die pH-Regelung des Reaktorinhalts.

### Definitionen:

*Biogas:* Unter Biogas ist ein Gas zu verstehen, das in einer anaeroben Fermentation in einer Anlage zur Erzeugung von Biogas unter Einwirkung von verschiedenen Mikroorganismen gebildet wird. Es enthält als Hauptbestandteile Methan und Kohlendioxid. Daneben enthält es Wasserdampf und gegebenenfalls kleine Anteile an Wasserstoff, Stickstoff, Sauerstoff, Schwefelwasserstoff und Ammoniak. Diese Art von Biogas wird auch als Rohbiogas bezeichnet und kann beispielsweise einem Blockheizkraftwerk zugeführt werden, um Strom und Wärme zu erzeugen. Wird das Rohbiogas weiter aufbereitet, um beispielsweise anschließend in ein Erdgasnetz eingespeist zu werden, so wird unter anderem Kohlendioxid abgetrennt. Das abgetrennte kohlendioxidreiche Gas aus einer Biogasaufbereitung kann bevorzugt als kohlendioxidhaltiges Eduktgas für die biologische Methanisierung verwendet werden. Allerdings ist auch Rohbiogas als kohlendioxidhaltiges Eduktgas für die biologische Methanisierung geeignet. Eine Anlage zur Erzeugung von Biogas kann beispielsweise eine Biogasanlage sein, in der das Biogas aus Biomasse erzeugt wird. Ferner kann die Anlage zur Erzeugung von Biogas auch eine Kläranlage sein, welche einen Faulturm aufweist. Das in einer Kläranlage erzeugte Biogas wird auch als Faulgas oder Klärgas bezeichnet. *Eduktgas:* Eduktgase für die Methanisierung sind wasserstoffhaltiges Gas und kohlendioxidhaltiges Gas bzw. Wasserstoff und Kohlendioxid.

*Methanangereichertes Gas* = *Produktgas* = *Biomethan:* Unter einem methanangereicherten Gas ist ein Gas zu verstehen, das durch die Einwirkung von hydrogenotrophen methanogenen Mikroorganismen in einem anaeroben Bioreaktor unter Zufuhr von wasserstoffhaltigem und kohlendioxidhaltigem Gas gebildet wird. Das methanangereicherte Gas ist gleichzeitig das Produktgas, das aus den Eduktgasen im Bioreaktor im Prozess der biologischen Methanisierung gebildet wird und den Bioreaktor in der Produktgasleitung verlässt. Das methanangereicherte Gas kann neben CH₄ auch Anteile aus den in dem Bioreaktor zur Methanisierung eingebrachten Eduktgasen, beispielsweise von nicht umgesetztem Wasserstoff und Kohlendioxid oder weiteren, in geringeren Mengen anwesenden Gasen wie Stickstoff, Schwefelwasserstoff oder Ammoniak enthalten, wie sie z.B. in einem Rohbiogas vorkommen, so dass das methanangereicherte Gas nicht zu 100 % aus Methan bestehen muss. Methan ist jedoch der Hauptbestandtei des methanangereicherten Gases. Das im Rahmen der vorliegenden Anmeldung hergestellte methanangereicherte Gas ist auch ein Biomethan und kann ebenso als methanangereichertes Biogas verstanden werden, wenn als kohlendioxidhaltiges Gas ein Rohbiogas eingesetzt wird. Der Begriff Biomethan ist als Abgrenzung zu synthetischem Methan zu verstehen, das bei der chemisch katalytischen Methanisierung gebildet wird.

*Bioerdgas und SNG (substitute natural gas):* Unter Bioerdgas wird ein Methangas verstanden, das nach den jeweils gültigen Richtlinien (in Deutschland beispielsweise DVGW-Richtlinien G260, G262) ins Erdgasnetz eingespeist werden kann und in einer biologischen Methanisierung unter Verwendung von CO₂ biogenen Ursprungs erzeugt wurde. Im Wesentlichen synonym verwendet wird der Begriff SNG, was als Abkürzung für "synthetic natural gas" steht, also ein auf technischem Wege hergestelltes Substitut für Erdgas, das wie dieses verwendet werden kann und als Austauschgas in ein Erdgasnetz eingespeist werden kann.

*Biomasse:* Unter Biomasse im Sinne der Erfindung werden alle Arten von vergärbaren nachwachsenden Rohstoffen wie Mais, Getreide, Gras, Silphie, Zuckerrüben, aber auch tierische Exkremente wie Rinder- oder Schweinegülle, Pferdemist oder Hühnertrockenkot, aber auch kommunales oder industrielles Abwasser sowie Bioabfälle oder organische Abfälle aus der Lebensmittelproduktion oder -verarbeitung sowie beliebige Mischungen aus diesen Gärsubstraten verstanden.

*Gärrest:* Als Gärrest oder Gärrückstand wird der Rückstand an Gärsubstrat bezeichnet, der bei der Vergärung von Biomasse in einer Anlage zur Erzeugung von Biogas zurückbleibt. Unter Gärrest wird beispielsweise der Rückstand nach der letzten temperierten Fermenterstufe (z.B. Nachgärer) in einer Biogasanlage verstanden. Ebenso wird unter Gärrest Faulschlamm aus Faultürmen von Kläranlagen verstanden.

*Bioreaktor* = *Methanisierungsreaktor:* Reaktor mit einem Methanisierungsmedium einschließlich methanogener Mikroorganismen, in dem aus Kohlendioxid und Wasserstoff das Produktgas Methan gebildet wird.

*Methanisierung:* Unter Methanisierung ist eine Methanbildung ausgehend von den gasförmigen Stoffen Wasserstoff und Kohlendioxid als Eduktgase zu verstehen. Biologische Methanisierung beschreibt die Bildung von Biomethan mit Hilfe von hydrogenotrophen methanogenen Mikroorganismen in einem wässrigen Medium. Sie erfolgt im Wesentlichen nach der chemischen Gleichung: 4 H₂ + CO₂ → CH₄ + 2 H₂O. Ist in einem der Eduktgase Kohlenmonoxid enthalten, kann die Methanbildung zusätzlich auch nach der Gleichung: 3 H₂ + CO → CH₄ + H₂O erfolgen. Andere Wege der Methanbildung beispielsweise aus Acetat oder Methylverbindungen können je nach verwendetem Methanisierungsmedium und verwendeten Reaktionsbedingungen nicht ausgeschlossen werden, spielen jedoch eine untergeordnete Rolle, da kohlendioxidhaltiges und wasserstoffhaltiges Gas in überwiegender Menge zugegeben wird.

*Methanisierungsmedium:* Bezeichnet den Reaktorinhalt des Bioreaktors, der geeignet ist, eine biologische Methanisierung durchzuführen. Das Methanisierungsmedium ist ein wässriges Reaktionsmedium und stellt das Nährmedium für die methanogenen Mikroorganismen dar. Es beinhaltet alle Nährstoffe und Spurenelemente und sonstigen Komponenten, die für das Wachstum entsprechender hydrogenotropher methanogener Mikroorganismen nötig sind. Als Methanisierungsmedium wird entweder ein synthetisches Kulturmedium für methanogene Mikroorganismen verwendet oder es wird ein komplexes Nährmedium auf der Grundlage von Biomasse eingesetzt, das im Wesentlichen aus einem Gärrest als Substrat besteht und gegebenenfalls durch Nährstoffe und/oder Spurenelemente ergänzt wird.

### Kurze Beschreibung der Zeichnungen

Die Erfindung soll nachfolgend anhand von Ausführungsbeispielen im Zusammenhang mit den Zeichnungen näher erläutert werden. Es versteht sich von selbst, dass die im Zusammenhang mit den Ausführungsbeispielen gemachten Angaben die Erfindung nicht beschränken sollen. Es zeigen
- Fig. 1: das erfindungsgemäße Verfahren in Form eines Blockdiagramms;
- Fig. 2: in grafischer Darstellung Messwerte der Produktgaszusammensetzung und verfahrenstechnischer Parameter bei Durchführung einer Ausführungsform des Verfahrens gemäß der vorliegenden Erfindung;
- Fig. 3: in grafischer Darstellung Messwerte der Produktgaszusammensetzung und verfahrenstechnischer Parameter bei Durchführung einer weiteren Ausführungsform des Verfahrens gemäß der vorliegenden Erfindung;
- Fig. 4A, 4B, 4C: in grafischer Darstellung Messwerte der Produktgaszusammensetzung und verfahrenstechnischer Parameter bei Durchführung einer weiteren Ausführungsform des Verfahrens gemäß der vorliegenden Erfindung;
- Fig. 5: in grafischer Darstellung Messwerte der Produktgaszusammensetzung und verfahrenstechnischer Parameter bei Durchführung einer weiteren Ausführungsform des Verfahrens gemäß der vorliegenden Erfindung.

### Wege zur Ausführung der Erfindung

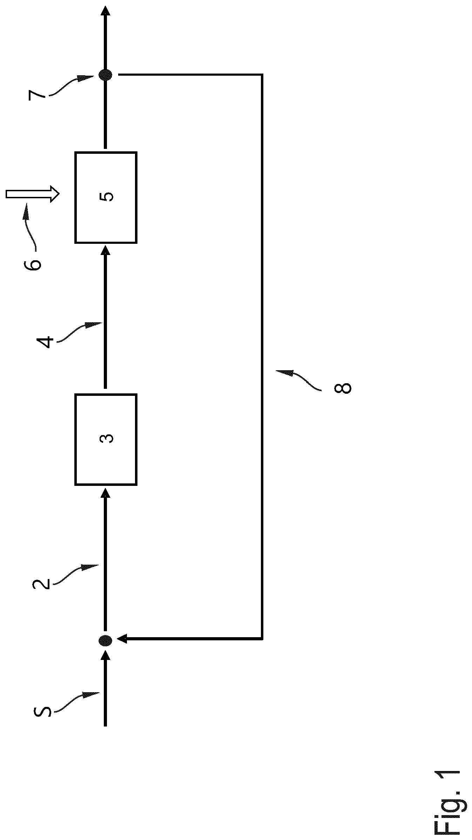

Das Ausführungsbeispiel 1 soll im Zusammenhang mit der Figur 1 näher erläutert werden. Die Figur 1 zeigt das erfindungsgemäße Verfahren in Form eines Blockdiagramms.

Zunächst erfolgt die Festlegung eines Sollwerts S für den Anteil an Kohlendioxid in dem aus dem Bioreaktor entnommenen methanangereicherten Gas. Die Regelung der Menge des zugeführten kohlendioxidhaltigen Gases und/oder die Regelung der Menge des zugeführten wasserstoffhaltigen Gases erfolgt erfindungsgemäß derart, dass der gemessene Istwert 7 des Anteils an CO₂ im Produktgas dem festgelegten Sollwert S möglichst nahe kommt oder diesem entspricht.

Die Regelung der Menge des zugeführten kohlendioxidhaltigen Gases und/oder die Regelung der Menge des zugeführten wasserstoffhaltigen Gases erfolgt ausschließlich auf Basis des Vergleichs von festgelegtem Sollwert S mit dem gemessenen Istwert 7 des Anteils an Kohlendioxid in dem aus dem Bioreaktor entnommenen methanangereicherten Gas. Die Regelabweichung 2 wird durch Bildung der Differenz von Sollwert S minus Istwert 7 berechnet.

Als Regler 3 fungiert ein entsprechend programmiertes Regelungsmodul, das üblicherweise auf einer Rechnereinheit installiert ist, die eine Rückführung 8 des gemessenen Istwerts 7 zum Startpunkt des Regelkreises vornimmt und dann einen Vergleich des festgelegten Sollwerts S mit dem gemessenen Istwert 7 durchführt. Ziel der Wirkungsweise des Reglers ist es, die Regelabweichung 2 auf Null zurückzuführen. Die Regelung der Menge des zugeführten kohlendioxidhaltigen Gases und/oder die Regelung der Menge des zugeführten wasserstoffhaltigen Gases wird derart durchgeführt, dass das Regelungsmodul ein verändertes Verhältnis der Eduktgase H₂ (bzw. wasserstoffhaltiges Gas) und CO₂ (bzw. CO₂-haltiges Gas) berechnet. Dieses dient als Stellgröße 4. Das Verhältnis der Eduktgase wird über die Zudosierung der Eduktgase in Reaktion auf den Istwert 7, also den CO₂-Gehalt im Produktgas geregelt.

Der Methanisierungsreaktor selbst stellt die Regelstrecke 5 des Systems dar. Als auf die Regelstrecke 5 einwirkende Störgrößen 6 können in dem System alle Parameter fungieren, die eine Änderung der Zusammensetzung des Produktgases der Methanisierung bewirken, also z.B. Änderungen des pH-Wertes oder der Temperatur im Bioreaktor, aber auch Änderungen in der Zusammensetzung des Reaktormediums aufgrund von Wachstum und Stoffwechsel der Mikroorganismen.

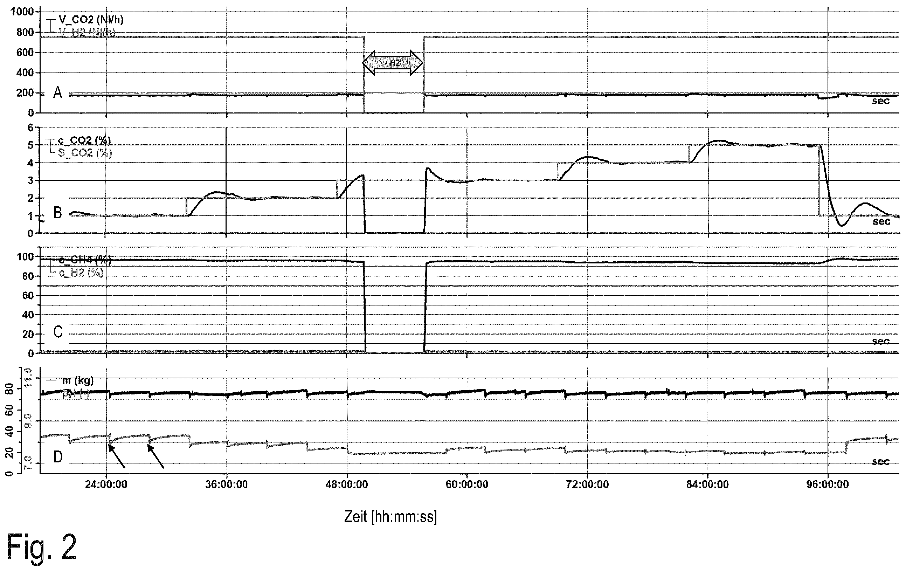

Die Figur 2 zeigt in grafischer Darstellung Messwerte der Produktgaszusammensetzung sowie weitere verfahrenstechnische Parameter bei Durchführung einer Ausführungsform des Verfahrens zur biologischen Methanisierung gemäß der vorliegenden Erfindung. Aus den Versuchsergebnissen geht hervor, dass bei Anwendung eines Verfahrens gemäß der vorliegenden Erfindung mit Sollwerten für den CO₂-Gehalt im Produktgas im Bereich von 1 Vol. % bis einschließlich 5 Vol. % ein Bioreaktor stabil betrieben werden kann, ohne dass eine pH-Regelung durch Messung des pH-Wertes im Reaktor und entsprechender Zugabe von Lauge oder Säure vorgenommen wird. Zudem zeigen die Daten, dass ohne weitere Gasaufbereitung ein methanangereichertes Gas als Produktgas erhalten wird, das einen sehr hohen Methangehalt von mindestens 93 Vol. % bei einer Wasserstoffkonzentration von weniger als 2,5 Vol. % aufweist.

Die biologische Methanisierung wurde in einem Bioreaktor durchgeführt, welcher Faulschlamm aus einer kommunalen Kläranlage, dem einzelne Spurenelemente und Nährstoffe zugesetzt wurden, als Methanisierungsmedium enthielt. Die biologische Methanisierung erfolgte unter Zusatz eines methanogenen Mikroorganismus aus der Gattung *Methanothermobacter.* Die Herstellung des methanangereicherten Gases wurde in einem Rührkesselreaktor mit 75 Liter Reaktorinhalt bei einer Temperatur von 65 °C und einem Druck von 7 bar durchgeführt. Über den gesamten Zeitraum wurde ein methanangereichertes Gas mit einer Methanbildungsrate (MBR) von 60 Normkubikmetern Methan pro Kubikmeter Reaktorinhalt pro Tag [Nm³/(m³d)] produziert. Es wurden über den in Figur 2 dargestellten Zeitraum CO₂-Regelungen mit Sollwerten von 1 Vol. %, 2 Vol. %, 3 Vol. %, 4 Vol. % und 5 Vol. % Kohlendioxid im Produktgas in aufsteigender Reihenfolge durchgeführt. Die Angabe auf der Zeitachse entsprechen einer Zeit in Stunden.

In Graph A der Figur 2 ist die Zugabe der Eduktgase H₂ und CO₂ dargestellt. Diese erfolgte aus Gasbündeln mit jeweils reinem H₂ und CO₂ in Druckgasflaschen. Der jeweilige Gasvolumenstrom in Normlitern pro Stunde [Nl/h] wurde über Massendurchflussregler der Firma Bronkhorst geregelt. Der Wasserstoffvolumenstrom (V_H2; graue Linie) wurde über den gesamten Versuchszeitraum konstant gehalten und mit 750 Nl/h vorgegeben. Der Wert für den zugeführten Kohlendioxidvolumenstrom (V_CO2; schwarze Linie) ergab sich nach Messung des Istwertes und der Berechnung der Regelabweichung aus der vom CO₂-Regler ausgegebenen Stellgröße in Verbindung mit dem vorgegebenen Wasserstoffvolumenstrom. Über den dargestellten Versuchszeitraum der Erhöhung der Sollwerte für CO₂ von 1 Vol. % bis 5 Vol. % ergaben sich Werte für den Kohlendioxidvolumenstrom im Bereich von 166 Ni/h bis 187 Nl/h. Im Versuchszeitraum von 50 Stunden bis 56 Stunden konnten aufgrund einer Störung der Wasserstoffzufuhr keine Eduktgase zugeführt werden, so dass auch kein methanangereichertes Gas produziert werden konnte.

In Graph B der Figur 2 ist die gemessene Konzentration an CO₂ im Produktgas (c_CO2; schwarze Linie) in Vol. % dargestellt. Diese entspricht dem Istwert der Regelgröße. Der im jeweiligen Zeitraum für das erfindungsgemäße Verfahren vorgegebene Sollwert an Kohlendioxid im Produktgas (S_CO2) in Vol. % ist durch eine graue Linie gezeigt.

Graph C der Figur 2 zeigt die gemessenen Werte für die Methankonzentration im Produktgas (c_CH4; schwarze Linie) in Vol. % sowie die Wasserstoffkonzentration im Produktgas (c_H2; graue Linie) in Vol. %. Alle Gaskomponenten des Produktgases wurden mit Inline-Gasanalysegeräten von Emerson gemessen.

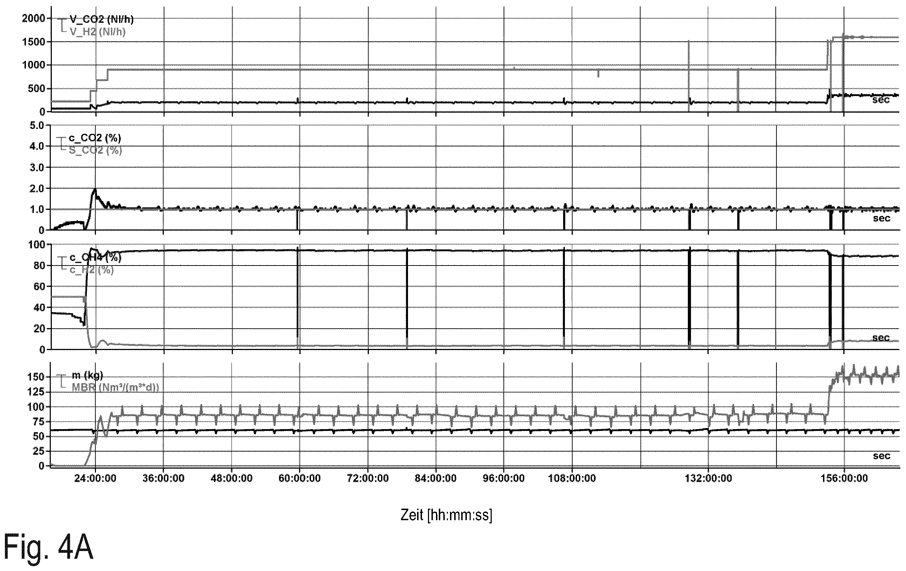

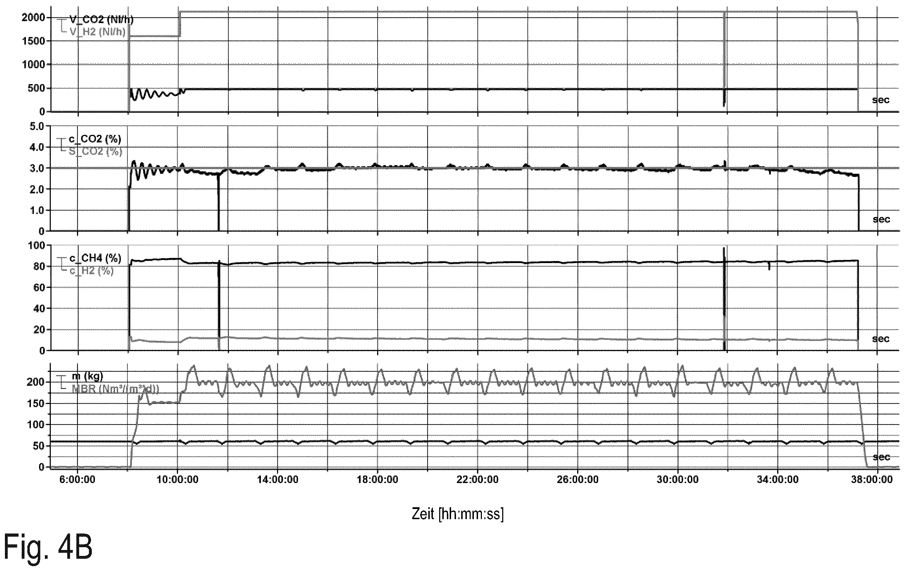

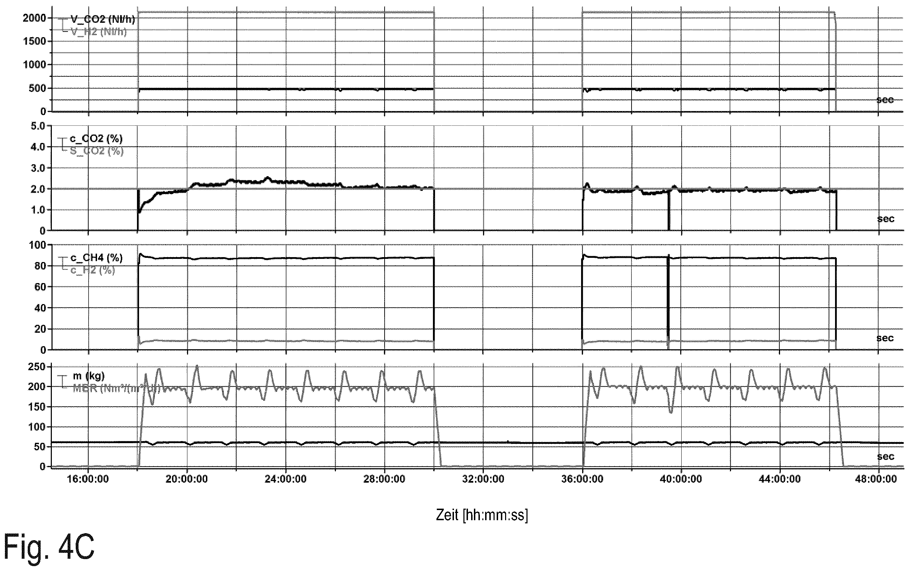

Graph D der Figur 2 zeigt die gemessenen Werte für die Masse des Reaktorinhalts (m; schwarze Linie) in Kilogram [kg] auf der links angegebenen Skala (schwarze Werte) sowie die gemessenen pH-Werte (pH; graue Linie) auf der rechts angegebenen Skala (graue Werte). Der Reaktorinhalt betrug im Mittel 75 Kilogramm. In regelmäßigen Abständen wurde ein Teil des Reaktorinhaltes abgelassen und anschließend wieder mit frischem Substrat bzw. Methanisierungsmedium ergänzt, so dass ein gewisser Austausch durch frisches Material erfolgte. Dies ist in Graph D der Figur 4 durch kleine Ausschläge nach unten und ein anschließendes Ansteigen der Masse erkennbar. Der pH-Wert des Methanisierungsmediums wurde nicht mit einer pH-Sonde direkt im Bioreaktor gemessen, sondern im Ablauf des Reaktors jeweils direkt nach dem Ablassen des auszutauschenden Reaktorinhalts. Der maßgebliche pH-Wert für den Reaktorinhalt entsprach jeweils dem niedrigsten gemessenen Wert in einem Austauschzyklus (exemplarisch gekennzeichnet durch Pfeile).

Über einen Zeitraum von jeweils etwa 12 Stunden wurde die biologische Methanisierung nach dem erfindungsgemäßen Verfahren einer CO₂-Regelung mit vorgegebenen Sollwerten von 1 Vol. %, 2 Vol. %, 3 Vol. %, 4 Vol. % und 5 Vol. % Kohlendioxid im Produktgas in aufsteigender Reihenfolge durchgeführt. Mit jedem der Sollwerte war eine stabile Methanisierung bei guter Gasqualität im Produktgas ohne Zugabe von Lauge oder Säure möglich. Es wurde eine Methanbildungsrate von 60 Normkubikmetern Methan pro Kubikmeter Reaktorinhalt pro Tag erreicht. Der vorgegebene Sollwert für die CO₂-Konzentration im Produktgas wurde jeweils nicht sofort nach der Eingabe des Wertes erreicht, sondern erst nach einer gewissen Anlaufzeit. Dieses Verhalten ist in gewissen Grenzen auch erwünscht, da sehr schnelle Änderungen der Reaktionsbedingungen in biologischen Systemen in der Regel nicht vorteilhaft sind, da sie die Stabilität von Prozessen gefährden können.

Auch ein gewisses Einschwingverhalten mit Werten über und unter dem vorgegebenen Sollwert wurde beobachtet. Dies ist jedoch ein regelungstypisches Verhalten und hängt in gewissen Grenzen von den eingestellten Regelungsparametern ab. In allen Fällen wurde jedoch nach wenigen Stunden der vorgegebene Sollwert auf einem stabilen Niveau erreicht und gehalten. Die Wasserstoffkonzentrationen im Produktgas waren durchweg kleiner als 2,5 Vol. %. Bis zu einer Sollkonzentration von 3 Vol. % CO₂ lag die CH₄-Konzentration bei größer oder gleich 95 Vol. %. Bei vorgegebenen Sollkonzentrationen von CO₂ von 4 Vol. % oder 5 Vol. % lag die CH₄-Konzentration bei größer oder gleich 93 Vol. %, da hier aufgrund der Vorgabe durch die Regelung bereits ein entsprechend hoher CO₂-Gehalt im Produktgas vorhanden war. Auch nach Unterbrechung der Methanisierung aufgrund einer Störung der Wasserstoffzufuhr im Versuchszeitraum von 50 bis 56 Stunden, konnte die Methanisierung ohne Verzögerung mit gleich hoher Methanbildungsrate und gleich guter Produktgasqualität wieder fortgeführt werden. Der pH-Wert, der in regelmäßigen Abständen jeweils direkt in dem aus dem Reaktor abgelassenen Reaktorinhalt bestimmt wurde, bewegte sich über den gesamten Versuchszeitraum in einem Bereich von etwa pH 8,1 bis 7,5.

Nach Einstellung des CO₂-Sollwertes auf 5 Vol. % wurde die Regelung in einem Schritt auf einen Sollwert von 1 Vol. % CO₂ heruntergefahren. Die Einschwingvorgänge wiesen zwar eine größere Amplitude auf als bei den Änderungen in Schritten von nur 1 Vol. %, auch führte die Regelung dazu, dass durch die Stellgröße der Volumenstrom für das Eduktgas CO₂ kurzfristig bis auf einen Wert von 147 Nl/h zurückgenommen wurde, jedoch wurde auch hier nach wenigen Stunden der vorgegebene Sollwert von 1 Vol. % CO₂ erreicht. Es gelang problemlos, den Bioreaktor samt Inhalt wieder auf den Ausgangszustand mit niedrigem CO₂-Gehalt zurückzuführen. Im Produktgas wurde mit einem eingestellten Sollwert für CO₂ von 1 Vol. % eine Methankonzentration von 97 Vol. % und eine Restwasserstoffkonzentration von 2 Vol. % erreicht. Dies erfüllt in der Regel die vorgegebenen Grenzen für ein einspeisefähiges Biomethan.

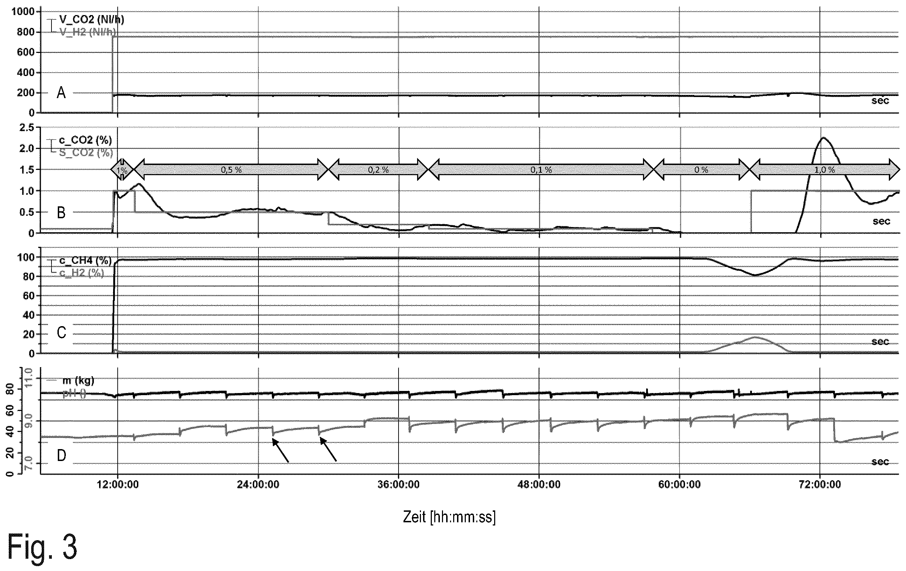

Die Figur 3 zeigt in grafischer Darstellung Messwerte der Produktgaszusammensetzung sowie weitere verfahrenstechnische Parameter bei Durchführung einer Ausführungsform des Verfahrens zur biologischen Methanisierung gemäß der vorliegenden Erfindung. Aus den Versuchsergebnissen geht hervor, dass bei Anwendung eines Verfahrens gemäß der vorliegenden Erfindung mit Sollwerten für den CO₂-Gehalt im Produktgas im Bereich von 1 Vol. % bis einschließlich 0,1 Vol. % ein Bioreaktor stabil betrieben werden kann, ohne dass eine pH-Regelung durch Messung des pH-Wertes im Reaktor und entsprechender Zugabe von Lauge oder Säure vorgenommen wird. Zudem zeigen die Daten, dass ohne weitere Gasaufbereitung ein methanangereichertes Gas als Produktgas erhalten wird, das einen sehr hohen Methangehalt von mindestens 97 Vol. % aufwies bei einer Wasserstoffkonzentration von weniger als 2 Vol. %.

Das in Figur 3 dargestellte Ausführungsbeispiel ist eine Weiterführung der in Figur 2 beschriebenen biologischen Methanisierung. Die Versuchsbedingungen sowie die Darstellung der Graphen A bis D entsprechen denen von Ausführungsbeispiel 2 mit dem Unterschied, dass im vorliegenden Fall CO₂-Sollwerte von 1 Vol. % bis hinab zu 0 Vol. % in den Schritten 1 Vol. %, 0,5 Vol. %, 0,2 Vol. %, 0,1 Vol. % und 0 Vol. % vorgegeben wurden.

Gestartet wurde in diesem Beispiel bei einem Sollwert von 1 Vol. % CO₂, gemessen im Produktgas. Diese Sollwertvorgabe entspricht dem Startwert und dem Endwert des Ausführungsbeispiels 2. Bis hinab zu einem Sollwert von 0,1 Vol. % CO₂ war eine biologische Methanisierung möglich. Die Werte für den Volumenstrom an Kohlendioxid für das Eduktgas lagen im Bereich von 178 Ni/h bis hin zu 167 Nl/h, während der Wasserstoffvolumenstrom auf 750 Nl/h konstant gehalten wurde. Die Methankonzentrationen im Produktgas lagen im Bereich zwischen 97 Vol. % und 98,5 Vol. % bei einer Wasserstoffkonzentration von ca. 1,5 Vol. % bis 1,75 Vol. %. Die gemessenen pH-Werte im Ablauf des Methanisierungsmediums aus dem Reaktor lagen zwischen pH 8,1 und pH 8,8. Die Einstellung auf einen Sollwert von 0,1 Vol. % CO₂ im Produktgas wurde für einen Zeitraum von 20 Stunden beibehalten.

Anschließend wurde so geregelt, dass im Produktgas kein CO₂ mehr gemessen werden konnte, indem der Sollwert für CO₂ auf Null gesetzt wurde. Diese Vorgabe für die CO₂ Regelung wurde über einen Zeitraum von 8 Stunden beibehalten. Wie aus Graph A der Figur 3 ersichtlich ist, ging dabei der Volumenstrom für das Eduktgas Kohlendioxid ausgehend von 171 Nl/h zurück bis auf eine Konzentration von 157 Nl/h. Der pH-Wert stieg an bis auf einen Wert von fast 9. Etwa zweieinhalb Stunden nach Vorgabe des Sollwertes von 0 Vol. % CO₂ im Produktgas war kein CO₂ mehr im Produktgas messbar. Nach weiteren 2 Stunden verschlechterte sich die Produktgasqualität erheblich. Der Methangehalt fiel in den nächsten 4 Stunden von einem Wert von 98 Vol. % auf einen Wert von 81 Vol. %. Gleichzeitig stieg der Wasserstoffanteil im Produktgas von etwa 1,5 Vol. % auf knapp 17 Vol. %.

Zusätzlich wurde gemessen, dass die Schwefelwasserstoffkonzentration im Produktgas ab einer Versuchszeit von 63 Stunden von einem Wert von 50 ppm auf einen Wert von über 120 ppm anstieg, einhergehend mit einem Anstieg der Wasserstoffkonzentration und einem Abfall der Methankonzentration. Ebenso konnte ein Anstieg der Ammoniakkonzentration im Produktgas beobachtet werden. Dieselben Beobachtungen wurden noch zwei weitere Male wiederholt als erneut in einem Versuchszeitraum Sollwerte von Null für die Konzentration von Kohlendioxid im Produktgas eingestellt wurden. Damit zeigt sich, dass eine Regelung in dem Sinne, dass das vorhandene Kohlendioxid vollständig verbraucht wird, bei der biologischen Methanisierung nicht funktioniert, während eine Regelung auf einen Sollwert für das CO₂ im Produktgas in einem Bereich von 0,1 Vol. % bis hin zu 5 Vol. % nach dem erfindungsgemäßen Verfahren funktioniert und ein methanangereichertes Gas von guter bis sehr guter Qualität liefert.

Als weiterer Vorteil des erfindungsgemäßen Verfahrens zeigt sich, dass überraschenderweise auf eine pH-Regelung durch Zugabe von Säure oder Lauge bei gleichzeitiger kontinuierlicher pH-Messung im Bioreaktor verzichtet werden konnte.

Um einen vollständigen "Absturz" der biologischen Methanisierung in dem Bioreaktor zu verhindern, wurde nach 66 Stunden Versuchszeit der Sollwert für das CO₂ im Produktgas von 0 Vol. % wieder auf 1 Vol. % erhöht. Während der Kohlendioxidfluss im Eduktgas aufgrund der Regelung direkt danach anstieg, verbesserte sich auch die Gasqualität schnell wieder. Bis zum Absinken des pH-Wertes und dem Einpendeln des CO₂ Gehalts im Produktgas auf 1 Vol. % dauerte es ein paar Stunden, jedoch konnte die biologische Methanisierung anschließend wieder fortgeführt werden und der Bioreaktor samt Inhalt in einen funktionsfähigen Zustand rückgeführt werden.

Die Figuren 4A, 4B, 4C, 4D zeigen ein weiteres Ausführungsbeispiel der Durchführung des Verfahrens zur biologischen Methanisierung gemäß der vorliegenden Erfindung. Die grafische Darstellung der Messwerte der Produktgaszusammensetzung sowie weiterer verfahrenstechnischer Parameter erfolgt analog zu der im Zusammenhang mit den Figuren 2 und 3 beschriebenen Weise mit dem einzigen Unterschied, dass in der untersten Teilabbildung anstelle des pH-Wertes jeweils die Methanbildungsrate (MBR) angegeben ist. Die regelmäßig wiederkehrenden Abweichungen nach unten und oben zu der angestrebten Methanbildungsrate ergeben sich dadurch, dass sich jeweils bei einem Substrataustausch im Bioreaktor über einen bestimmten Zeitraum Änderungen in der Menge des Reaktorinhalts ergeben, sowie Druckschwankungen, die zu vorübergehenden Änderungen des Volumenstroms des Produktgases führen, welche rechnerisch zu Veränderungen in der MBR führen. Der pH-Wert wurde nicht im Reaktor gemessen, sondern es wurden wiederholt Proben genommen, um den pH-Wert zu messen.

Die biologische Methanisierung wurde in einem Bioreaktor durchgeführt, welcher Faulschlamm aus einer kommunalen Kläranlage enthielt. In diesem Beispiel wurden jedoch keine Spurenelemente und Nährstoffe zugesetzt, lediglich ein Reduktionsmittel wurde dem Methanisierungsmedium zugegeben. Die biologische Methanisierung erfolgte ohne Zusatz eines methanogenen Mikroorganismus. Die Herstellung des methanangereicherten Gases wurde in einem Rührkesselreaktor mit 60 Liter Reaktorinhalt bei einer Temperatur von 65 °C und einem Druck von 7 bar durchgeführt.

In dem in Figur 4A dargestellten Zeitraum wurde der Sollwert für die CO₂ Konzentration im Produktgas für die Regelung auf 1 Vol. % eingestellt. Beim Start der Methanisierung wurde der Gasvolumenstrom für das Eduktgas Wasserstoff schrittweise angehoben, so dass nach 3 Stunden bereits methanangereichertes Gas mit einer Methanbildungsrate von 85 Nm³/(m³d) produziert werden konnte. Zu diesem Zeitpunkt hatte sich der CO₂ Anteil im Produktgas auch bereits stabil auf den Sollwert von 1 Vol. % eingependelt. Über den gesamten Zeitraum von Versuchszeit 30 Stunden bis hin zu 150 Stunden verlief die Methansisierung stabil mit derselben Methanbildungsrate. Es wurde ein methanangereichertes Gas produziert mit einem Methananteil von etwa 94 Vol. % bis 95 Vol. %, einem Wasserstoffanteil von etwa 3,5 Vol. % und einem Kohlendioxidanteil von 1 Vol. %. Die Störsignale, die sich durch unregelmäßig wiederkehrende senkrechte Linien in den Werten der Gasanalyse bemerkbar machen, rühren von Probenahmen her. Ab dem Versuchszeitpunkt 152 Stunden wurde die Eduktgaszufuhr gesteigert, so dass nach weiteren 2 bis 3 Stunden eine Methanbildungsrate von 153 Nm³/(m³d) erreicht wurde. Der Sollwert für die CO₂ Regelung wurde hierbei nicht verändert. Bei der Methanbildungsrate von 153 Nm³/(m³d) wurde eine Gasqualität mit etwa 89 Vol. % Methan und ca. 8 Vol. % Wasserstoff erreicht.

Nach einer Pause in der Eduktgaszufuhr wurde die Methansierung mit derselben Methanbildungsrate fortgeführt, jedoch unter Vorgabe eines CO₂ Sollwertes von 3 Vol. %. Dies ist in Figur 4B dargestellt. Die Volumenströme für H₂ und CO₂ wurden so vorgegeben, dass von Anfang an eine Methanbildungrate von 153 Nm³/(m³d) erreicht wurde. Das Einschwingen des CO₂ Wertes im Produktgas auf 3 Vol. % dauerte ca. 4 h.

Das methanangereicherte Gas enthielt ca. 87 Vol. % Methan und ca. 8 Vol. % Wasserstoff sowie 3 Vol. % Kohlendioxid. Direkt im Anschluss wurde die Methanbildungsrate unter Beibehaltung des CO₂ Sollwerts auf einen Wert von 200 Nm³/(m³d) erhöht. Dies war problemlos möglich. Die Gasqualität verschlechterte sich bei der hohen Methanbildungsrate etwas auf Werte von ca. 84 Vol. % Methan und ca. 11 Vol. % Wasserstoff.

Nach einer längeren Methanisierungspause wurde die Methansierung mit einer Methanbildungsrate von 200 Nm³/(m³d) fortgesetzt, wobei der CO₂ Sollwert auf einen Wert von 2 % eingestellt wurde. Dies ist in Figur 4C dargestellt. Beim Methanisierungsstart bei Versuchszeit 18 Stunden dauerte es 8 Stunden bis der CO₂-Wert von 2 Vol. % stabil erreicht wurde. Die Gasqualität für des Produktgas verbesserte sich zu etwa 87,5 Vol. % Methan und 8,5 Vol. % Wasserstoff. Nach einer Unterbrechung der Eduktgaszufuhr von 6 Stunden gelang ein sehr schnelles Wiederanfahren unter denselben Bedingungen, wobei auch der CO₂ Sollwert von 2 Vol. % von Anfang an erreicht wurde.

Das in den Figuren 4A bis 4D beschriebene Ausführungsbeispiel zeigt, dass in einem weiteren Methanisierungsmedium auch bei hohen Methanbildungsraten bis 200 Nm³/(m³d) eine biologische Methanisierung gemäß dem Verfahren der vorliegenden Erfindung bei verschiedenen vorgegebenen Sollwerten für das Kohlendioxid im Produktgas möglich war, auch wenn das methanangereicherte Gas in diesem Fall direkt nach dem Verlassen des Bioreaktors noch keine Einspeisequalität aufwies. Durch eine anschließende Gasaufbereitung mit Abtrennung des Restwasserstoffs kann das so hergestellte methanangereicherte Gas in ein Bioerdgas bzw. SNG umgewandelt werden. Auch in diesem Beispiel erfolgte keine pH-Regelung durch Zugabe von Säure oder Lauge. Der gemessene pH-Wert in den Proben aus dem Bioreaktor lag im gesamten Versuchszeitraum bei Werten zwischen pH 7,6 und 8,4, welche für die Methanbildung gut geeignet waren. Auch die gemessenen Werte für den Schwefelwasserstoffgehalt oder Ammoniakgehalt im Produktgas waren unkritisch.

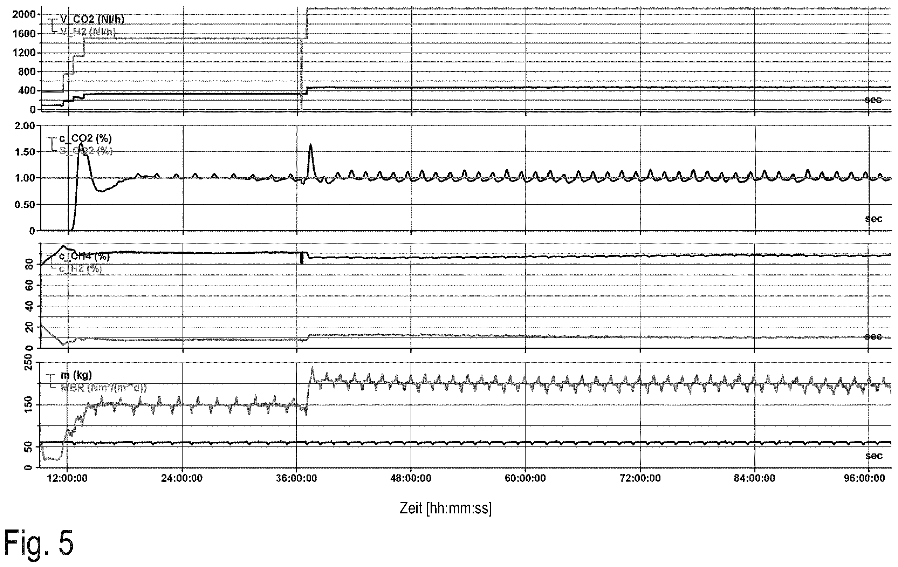

Die Figur 5 zeigt ein weiteres Ausführungsbeispiel der Durchführung des Verfahrens zur biologischen Methanisierung gemäß der vorliegenden Erfindung. Die grafische Darstellung der Messwerte der Produktgaszusammensetzung sowie weiterer verfahrenstechnischer Parameter erfolgt analog zu der im Zusammenhang mit den Figuren 4A bis 4D beschriebenen Weise.

Die biologische Methanisierung wurde in einem Bioreaktor durchgeführt, welcher als Methanisierungsmedium ein synthetisches Kulturmedium enthielt, wie es im Stand der Technik für hydrogenotrophe methanogene Archaeen verwendet wird (siehe beispielsweise WO 2008/094282 A1 oder WO 2012/110257 A1). Das Medium wurde beimpft mit einem methanogenen Stamm aus der Ordnung *Methanobacteriales.* Die Herstellung des methanangereicherten Gases wurde in einem Rührkesselreaktor mit 60 Liter Reaktorinhalt bei einer Temperatur von 65 °C und einem Druck von 7 bar durchgeführt.

In dem in Figur 5 dargestellten Zeitraum wurde der Sollwert für die CO₂ Konzentration im Produktgas für die Regelung auf 1 Vol. % eingestellt. Zum Zeitpunkt Null wurde die biologische Methanisierung gestartet. Dabei wurde der Gasvolumenstrom für das Eduktgas Wasserstoff schrittweise angehoben, so dass nach 4 Stunden bereits methanangereichertes Gas mit einer Methanbildungsrate von 150 Nm³/(m³d) produziert werden konnte. Nach weiteren 5 Stunden hatte sich der CO₂ Anteil im Produktgas stabil auf den Sollwert von 1 Vol. % eingependelt. Bis zu einer Versuchszeit von 37 Stunden wurde ein methanangereichertes Gas mit einem Methangehalt von 90 Vol. % bis 92 Vol. % produziert, bei einem Wasserstoffgehalt von 7 Vol. % bis 8,5 Vol. %. Ab dem Versuchszeitpunkt 37 Stunden wurde die Eduktgaszufuhr gesteigert, so dass kurze Zeit später eine Methanbildungsrate von 200 Nm³/(m³d) erreicht wurde. Der Sollwert für die CO₂ Regelung wurde hierbei nicht verändert.

Über den gesamten weiteren Versuchszeitraum verlief die Methansisierung sehr stabil, wobei sich die Gasqualität über die Versuchszeit leicht verbesserte. Es wurde ein methanangereichertes Gas produziert, bei dem sich der Methananteil von etwa 85 Vol. % bis etwa 89 Vol. % langsam erhöhte, während der Wasserstoffanteil von etwa 13,5 Vol. % auf etwa 10 Vol. % sank und der Kohlendioxidanteil kontinuierlich um den Sollwert von 1 Vol. % pendelte. Die in diesem Ausführungsbeispiel mit einem System, in dem ein synthetisches Kulturmedium mit definiertem Methanogenen als Reaktorinhalt verwendet wurde, erzielten Gasqualitäten im Produktgas, stimmen bei vergleichbaren Methanbildungsraten sehr gut mit den in Ausführungsbeispiel 4 erzielten Werten überein, in dem Faulschlamm als Substrat verwendet wurde und kein definierter methanogener Mikroorganismus zugegeben wurde.

Nach dem Start der Methanisierung musste sich der pH-Wert beim dem synthetischen Kulturmedium erst etwas stabilisieren, aber ab einem Zeitpunkt von etwa 30 Stunden wurde nur noch in regelmäßigen Abständen eine geringe Menge Natronlauge zudosiert, um den pH-Wert auf einem Wert zwischen pH 6,6 und pH 7,2 zu halten, in dem die biologische Methanisierung einwandfrei funktionierte, obwohl nur nach dem CO₂ Anteil im Produktgas, wie in der vorliegenden Erfindung beschrieben, geregelt wurde.

### Bezugszeichenliste

- S: Sollwert (Führungsgröße)
- 2: Regelabweichung (Sollwert - Istwert)
- 3: Regler
- 4: Stellgröße
- 5: Regelstrecke
- 6: Störgröße
- 7: Istwert (Regelgröße)
- 8: Rückführung

## Patentansprüche

1. Verfahren zur Erzeugung eines methanangereicherten Gases umfassend die Schritte
a) Bereitstellen eines Bioreaktors aufweisend
- zumindest eine Vorrichtung für die Zufuhr eines Gases,
- zumindest einen Auslass für die Entnahme des in dem Bioreaktor entstehenden methanangereicherten Gases,
b) Bereitstellen einer Vorrichtung zur Bestimmung des Anteils an Kohlendioxid in dem aus dem Bioreaktor entnommenen methanangereicherten Gas,
c) Festlegung eines Sollwerts S für den Anteil an Kohlendioxid in dem aus dem Bioreaktor entnommenen methanangereicherten Gas,
d) Zufuhr von kohlendioxidhaltigem Gas in den Bioreaktor,
e) Zufuhr von wasserstoffhaltigem Gas in den Bioreaktor,
f) Bildung von methanangereichertem Gas in dem Bioreaktor,
g) Entnahme des in dem Bioreaktor gebildeten methanangereicherten Gases aus dem Bioreaktor,
h) Bestimmen eines Istwertes für den Anteil an Kohlendioxid in dem aus dem Bioreaktor entnommenen methanangereicherten Gas,
i) Vergleich des in Schritt c) festgelegten Sollwerts S mit dem in Schritt h) bestimmten Istwert,
j) Regelung der Menge des in Schritt d) zugeführten kohlendioxidhaltigen Gases und/oder Regelung der Menge des in Schritt e) zugeführten wasserstoffhaltigen Gases derart, dass der in Schritt h) bestimmte Istwert dem in Schritt c) festgelegten Sollwert S entspricht,
**dadurch gekennzeichnet, dass** der in Schritt c) festgelegte Sollwert S für den Anteil an Kohlendioxid in dem aus dem Bioreaktor entnommenen methanangereicherten Gas der Bedingung 0 Vol. % < S ≤ 5 Vol. % genügt.

2. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0 Vol. % < S ≤ 4 Vol. % genügt.

3. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0 Vol. % < S ≤ 2 Vol. % genügt.

4. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0 Vol. % < S ≤ 1,5 Vol. % genügt.

5. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0,1 Vol. % ≤ S ≤ 5 Vol. % genügt.

6. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0,1 Vol. % ≤ S ≤ 4 Vol. % genügt.

7. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0,1 Vol. % ≤ S ≤ 2 Vol. % genügt.

8. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0,1 Vol. % ≤ S ≤ 1,5 Vol. % genügt.

9. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0,5 Vol. % ≤ S ≤ 5 Vol. % genügt.

10. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0,5 Vol. % ≤ S ≤ 4 Vol. % genügt.

11. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0,5 Vol. % ≤ S ≤ 2 Vol. % genügt.

12. Verfahren nach Anspruch 1, **dadurch gekennzeichnet, dass** der in Schritt c) festgelegte Sollwert S für den Anteil an Kohlendioxid in dem aus dem Bioreaktor entnommenen methanangereicherten Gas der Bedingung 0,5 Vol. % ≤ S ≤ 1,5 Vol. % genügt.

13. Verfahren nach einem der Ansprüche 1 bis 12, **dadurch gekennzeichnet, dass** zumindest zwei Vorrichtungen für die Zufuhr von Gasen vorgesehen sind, wobei es sich um zumindest eine Vorrichtung für die Zufuhr eines kohlendioxidhaltigen Gases und zumindest eine Vorrichtung für die Zufuhr eines wasserstoffhaltigen Gases handelt.

14. Verfahren nach einem der Ansprüche 1 bis 13, **dadurch gekennzeichnet, dass** es sich bei der Vorrichtung für die Zufuhr eines kohlendioxidhaltigen Gases und/oder bei der Vorrichtung für die Zufuhr eines wasserstoffhaltigen Gases um eine Vorrichtung zur Durchflussregelung handelt.

15. Verfahren nach einem der Ansprüche 1 bis 14, **dadurch gekennzeichnet, dass** die Regelung in Schritt j) ausschließlich auf Basis des in Schritt i) durchgeführten Vergleichs erfolgt.

16. Verfahren nach einem der Ansprüche 1 bis 15, **dadurch gekennzeichnet, dass** der Vergleich in Schritt i) und die Regelung in Schritt j) durch eine Rechnereinheit durchgeführt werden.

## Claims

1. Method for producing a methane-enriched gas, comprising the steps of
a) providing a bioreactor having
- at least one device for supplying a gas,
- at least one outlet for removing the methane-enriched gas generated in the bioreactor,
b) providing a device for determining the proportion of carbon dioxide in the methane-enriched gas removed from the bioreactor,
c) specifying a nominal value S for the proportion of carbon dioxide in the methane-enriched gas removed from the bioreactor,
d) supplying carbon dioxide-containing gas to the bioreactor,
e) supplying hydrogen-containing gas to the bioreactor,
f) forming methane-enriched gas in the bioreactor,
g) removing the methane-enriched gas formed in the bioreactor from the bioreactor,
h) determining an actual value for the proportion of carbon dioxide in the methane-enriched gas removed from the bioreactor,
i) comparing the nominal value S specified in step c) with the actual value determined in step h),
j) regulating the quantity of carbon dioxide-containing gas supplied in step d) and/or regulating the quantity of hydrogen-containing gas supplied in step e) in a manner such that the actual value determined in step h) corresponds to the nominal value S specified in step c),
**characterized in that** the nominal value S specified in step c) for the proportion of carbon dioxide in the methane-enriched gas removed from the bioreactor satisfies the condition 0 vol% < S ≤ 5 vol%.

2. Method according to claim 1, **characterized in that** the nominal value S specified in step c) for the proportion of carbon dioxide in the methane-enriched gas removed from the bioreactor satisfies the condition 0 vol% < S ≤ 4 vol%.

3. Method according to claim 1, **characterized in that** the nominal value S specified in step c) for the proportion of carbon dioxide in the methane-enriched gas removed from the bioreactor satisfies the condition 0 vol% < S ≤ 2 vol%.

4. Method according to claim 1, **characterized in that** the nominal value S specified in step c) for the proportion of carbon dioxide in the methane-enriched gas removed from the bioreactor satisfies the condition 0 vol% < S ≤ 1.5 vol%.

5. Method according to claim 1, **characterized in that** the nominal value S specified in step c) for the proportion of carbon dioxide in the methane-enriched gas removed from the bioreactor satisfies the condition 0.1 vol% ≤ S ≤ 5 vol%.

6. Method according to claim 1, **characterized in that** the nominal value S specified in step c) for the proportion of carbon dioxide in the methane-enriched gas removed from the bioreactor satisfies the condition 0.1 vol% ≤ S ≤ 4 vol%.

7. Method according to claim 1, **characterized in that** the nominal value S specified in step c) for the proportion of carbon dioxide in the methane-enriched gas removed from the bioreactor satisfies the condition 0.1 vol% ≤ S ≤ 2 vol%.

8. Method according to claim 1, **characterized in that** the nominal value S specified in step c) for the proportion of carbon dioxide in the methane-enriched gas removed from the bioreactor satisfies the condition 0.1 vol% ≤ S ≤ 1.5 vol%.

9. Method according to claim 1, **characterized in that** the nominal value S specified in step c) for the proportion of carbon dioxide in the methane-enriched gas removed from the bioreactor satisfies the condition 0.5 vol% ≤ S ≤ 5 vol%.

10. Method according to claim 1, **characterized in that** the nominal value S specified in step c) for the proportion of carbon dioxide in the methane-enriched gas removed from the bioreactor satisfies the condition 0.5 vol% ≤ S ≤ 4 vol%.

11. Method according to claim 1, **characterized in that** the nominal value S specified in step c) for the proportion of carbon dioxide in the methane-enriched gas removed from the bioreactor satisfies the condition 0.5 vol% ≤ S ≤ 2 vol%.

12. Method according to claim 1, **characterized in that** the nominal value S specified in step c) for the proportion of carbon dioxide in the methane-enriched gas removed from the bioreactor satisfies the condition 0.5 vol% ≤ S ≤ 1.5 vol%.

13. Method according to one of claims 1 to 12, **characterized in that** at least two devices are provided for supplying gases, wherein these are at least one device for supplying a carbon dioxide-containing gas and at least one device for supplying a hydrogen-containing gas.

14. Method according to one of claims 1 to 13, **characterized in that** the device for supplying a carbon dioxide-containing gas and/or the device for supplying a hydrogen-containing gas is/are a flow control device.

15. Method according to one of claims 1 to 14, **characterized in that** the regulation in step j) is carried out exclusively on the basis of the comparison carried out in step i).

16. Method according to one of claims 1 to 15, **characterized in that** the comparison in step i) and the regulation in step j) are carried out by means of a processing unit.

## Revendications

1. Procédé de production d'un gaz enrichi en méthane, comprenant les étapes consistant à
a) mettre à disposition un bioréacteur comportant
- au moins un dispositif destiné à introduire un gaz,
- au moins une sortie destinée à prélever le gaz enrichi en méthane qui est produit dans ledit bioréacteur,
b) mettre à disposition un dispositif destiné à déterminer la proportion de dioxyde de carbone dans le gaz enrichi en méthane qui a été prélevé dudit bioréacteur,
c) fixer une consigne S pour la proportion de dioxyde de carbone dans le gaz enrichi en méthane qui a été prélevé dudit bioréacteur,
d) introduire dans ledit bioréacteur du gaz contenant du dioxyde de carbone,
e) introduire dans ledit bioréacteur du gaz contenant de l'hydrogène,
f) former, au sein dudit bioréacteur, du gaz enrichi en méthane,
g) prélever, dudit bioréacteur, le gaz enrichi en méthane qui a été formé dans le bioréacteur,
h) déterminer la valeur réelle de la proportion de dioxyde de carbone dans le gaz enrichi en méthane qui a été prélevé dudit bioréacteur,
i) comparer la consigne S fixée à l'étape c) avec la valeur réelle déterminée à l'étape h),
j) régler la quantité du gaz contenant du dioxyde de carbone qui est introduit à l'étape d) et/ou régler la quantité du gaz contenant de l'hydrogène qui est introduit à l'étape h), de manière à ce que la valeur réelle déterminée à l'étape h) corresponde à la consigne S fixée à l'étape c),
**caractérisé en ce que** la consigne S fixée à l'étape c) pour la proportion de dioxyde de carbone dans le gaz enrichi en méthane qui est prélevé dudit bioréacteur satisfait à la condition 0 % en vol. < S ≤ 5 % en vol.

2. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0 % en vol. < S ≤ 4 % en vol.

3. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0 % en vol. < S ≤ 2 % en vol.

4. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0 % en vol. < S ≤ 1,5 % en vol.

5. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0,1 % en vol. ≤ S ≤ 5 en vol.

6. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0,1 % en vol. ≤ S ≤ 4 % en vol.

7. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0,1 % en vol. ≤ S ≤ 2 % en vol.

8. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0,1 % en vol. ≤ S ≤ 1,5 % en vol.

9. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0,5 % en vol. ≤ S ≤ 5 % en vol.

10. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0,5 % en vol. ≤ S ≤ 4 % en vol.

11. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0,5 % en vol. ≤ S ≤ 2 en vol.

12. Procédé selon la revendication 1, **caractérisé en ce que** la consigne S fixée à l'étape c) pour la proportion de dioxyde de carbone dans le gaz enrichi en méthane qui est prélevé dudit bioréacteur satisfait à la condition 0,5 % en vol. ≤ S ≤ 1,5 % en vol.

13. Procédé selon l'une des revendications 1 à 12, **caractérisé en ce qu'**il implique au moins deux dispositifs destinés à introduire des gaz, s'agissant d'au moins un dispositif destiné à introduire un gaz contenant du dioxyde de carbone et d'au moins un dispositif destiné à introduire un gaz contenant de l'hydrogène.

14. Procédé selon l'une des revendications 1 à 13, **caractérisé en ce que** le dispositif destiné à introduire un gaz contenant du dioxyde de carbone et/ou le dispositif destiné à introduire un gaz contenant de l'hydrogène correspondent à un dispositif permettant de régler le débit.

15. Procédé selon l'une des revendications 1 à 14, **caractérisé en ce que** le réglage à l'étape j) est effectué exclusivement sur la base de la comparaison réalisée à l'étape i).

16. Procédé selon l'une des revendications 1 à 15, **caractérisé en ce que** la comparaison à l'étape i) et le réglage à l'étape j) sont réalisés par un ordinateur.
